# EUROPÄISCHE PATENTSCHRIFT

(11) **EP 2 247 274 B1**
(45) Veröffentlichungstag und Bekanntmachung des Hinweises auf die Patenterteilung: **26.10.2016**
(21) Anmeldenummer: 09705481.1
(22) Anmeldetag: 30.01.2009
(51) Int. Cl.: A61J 1/14, B65D 51/00

(54) **Verschlusskappe für Behälter**
Closure cap for containers
Bouchon de fermeture pour récipients

(30) Priorität: 01.02.2008 DE 102008007252
(43) Veröffentlichungstag der Anmeldung: 10.11.2010
(73) Patentinhaber: Plümat Plate & Lübeck GmbH & Co., 32339 Espelkamp (DE)
(72) Erfinder: PULLER, Stefan, 32339 Espelkamp (DE)
(74) Vertreter: Harrison, Robert John
(86) Internationale Anmeldenummer: PCT/EP2009/051092
(87) Internationale Veröffentlichungsnummer: WO 2009/095488

(56) Entgegenhaltungen:
- EP-A1- 1 384 466
- WO-A-01/41698
- DE-A1- 10 336 523
- DE-U- 1 856 621
- US-A1- 2003 052 074

## Beschreibung

### Gebiet der Erfindung

Die Erfindung betrifft eine Verschlusskappe für einen medizinischen Behälter z.B. einen Beutel oder eine Flasche, die einen Zuspritz- und einen Entnahmeport aufweist.

### Hintergrund und Stand der Technik

Die vorliegende Erfindung betrifft eine Verschlusskappe für einen medizinischen Behälter, z.B. einen Beutel oder eine Flasche, wie er in Zusammenhang mit Infusionen, Nährlösungen und dergleichen verwendet wird, die Patienten verabreicht werden. Medizinische Behälter sind üblicherweise aus flexiblem Material ausgeführt, z.B. aus PE-Folie. Alternativ können medizinische Behälter aus festem Material ausgeführt sein, z.B. aus Glas, festem Kunststoff oder dergleichen. Die Verschlusskappe gemäß der vorliegenden Erfindung umfasst einen Zuspritzport zur Zugabe von Substanzen wie etwa Medikamenten zum flüssigen Inhalt des Behälters. Ferner verfügt die Verschlusskappe über einen Entnahmeport zur Verabreichung des Inhalts des Behälters.

Für die Verwendung der Verschlusskappe gemäß der vorliegenden Erfindung ergeben sich spezifische Anforderungen bei Verwendung im medizinisch-klinischen Bereich. So muss der Inhalt des Behälters durch den Behälter und die Verschlusskappe steril gehalten werden. Es darf kein offener Kontakt zwischen dem Inhalt des Behälters und der Umwelt bestehen, da der Inhalt sonst verunreinigt werden könnte. Es ist ausschließlich ein kontrollierter, steriler Kontakt vom Inneren des Behälters nach außerhalb des Behälters zulässig. Dies kann entweder bei der Zugabe von Substanzen zum Beutelinhalt geschehen, z. B. unter Verwendung steriler Kanülen und Nadeln, die in den Zuspritzport eingeführt werden oder bei Entnahme des Inhalts aus dem Behälter über den Entnahmeport, wobei sichergestellt sein muss, dass sterile Bedingungen bis ins Innere des Körpers des Patienten, z.B. bis in eine Vene des Patienten gegeben sind. Daher muss der Zuspritzport nach Zugabe der Substanz zuverlässig wieder verschlossen werden können. Bei der Entnahme von Inhalt aus dem Behälter darf es zu keinen Undichtigkeiten am Entnahmeport kommen, da sonst Inhalt aus dem Behälter verloren ginge oder der Inhalt kontaminiert werden könnte.

Beides ist für medizinische Anwendungen unzulässig, da einerseits nicht mehr die gewünschte Menge an Inhalt aus dem Behälter verabreicht wird und andererseits für den Patienten ein Infektionsrisikö besteht. Darüber hinaus muss die Unversehrtheit des Behälters nach außen sichtbar sein. Man muss also zweifelsfrei erkennen können, ob Substanzen zum ursprünglichen Inhalt des Behälters über den Zuspritzport zugegeben wurden oder nicht. Gleichzeitig ist es erforderlich, zweifelsfrei zu erkennen, ob aus dem Behälter bereits Inhalt entnommen wurde oder nicht. Üblicherweise wird der Entnahmeport eines medizinischen Behälters über einen Spike geöffnet. Der eingeführte Spike bleibt bis zur Leerung des Behälters im Entnahmeport.

Es existieren unterschiedliche Normen und Ausführungsformen für Spikes. Diese unterscheiden sich hinsichtlich ihrer Größe und Geometrie. Es kann also notwendig sein, für einen bestimmten medizinischen Behälter erst den passenden Spike zu finden.

Üblicherweise werden Entnahmeports eines medizinischen Beutels über Gummischeiben abgedichtet. Die Gummischeiben können sich in bestimmten Grenzen an die unterschiedlichen Durchmesser der Spikes anpassen und so Differenzen in der Geometrie unterschiedlicher Spikes, die z. T. länderspezifisch sind, ausgleichen. Ein Entnahmeport, der international zum Einsatz kommen soll, muss sich also in gewissen Grenzen an die unterschiedlichen Geometrien unterschiedlicher Spikes anpassen können.

Ein Vorteil des Entnahmeports gemäß der vorliegenden Erfindung liegt ferner darin, dass konstruktionsbedingt ohne Verwendung einer teuren Gummischeibe auch verschiedene Spikedurchmesser verwendet werden können. Eine Wiederabdichtungsfunktion ist dabei zwangsläufig nicht mehr gegeben. Dies ist aber auch nicht unbedingt erforderlich, da sich im Markt sowohl Systeme mit als auch ohne Wiederabdichtungsfunktion befinden.

Im Markt befindliche Verschlüsse z.B. der sog. Twist-Off Stopfen für Schläuche, der sehr weite Verbreitung gefunden hat, sind ebenfalls ohne Gummischeibe aber mit einer Trennmembran im Entnahmeport ausgeführt.

Als Trennmembran bzw. Membran soll im Zusammenhang mit dieser Offenbarung folgendes verstanden werden: Ganz allgemein liefert eine Membran eine Trennung von Beutelinhalt und Umwelt. Ferner kann unter einer Membran eine Trennung zwischen Beutelinhalt und einem Dichtungselement, z.B. einer Gummischeibe im Zuspritzport verstanden werden. Im Sinne dieser Definition umfasst die erfindungsgemäße Verschlusskappe zwei Membranen; jeweils eine im Entnahmeport und eine im Zuspritzport.

Für den Twist-Off Stopfen erfolgt das Halten des Spikes sowie das Abdichten auf dem Konus des Spikes durch die Flexibilität des Dichtungselements, das aus sehr weichem Material gebildet ist. Der Twist-Off Stopfen ermöglicht also heute bereits die Verwendung unterschiedlicher Spikegeometrien.

Bei der erfindungsgemäßen Verschlusskappe muss dagegen ein hartes Granulat gewählt werden, um die nötige Stabilität der Kappe zu erreichen. Wird hartes Granulat verwendet, so funktioniert das übliche Halten und Abdichten am Spike nicht mehr. Die erfindungsgemäße Verschlusskappe ermöglicht ein Halten und Abdichten des Spikes ohne dass dazu, wie bisher üblich, weiche Materialien in Form eines Stopfens oder einer Gummischeibe für Dichtungszwecke erforderlich sind.

Es existiert eine Vielzahl verschiedener Verschlusskappen für medizinische Behälter. So beschreibt das Europäische Patent EP 0766955 der Firma B. Braun Melsungen AG ein einstückig ausgeführtes Zuspritz- und Entnahmesystem für medizinische Beutel, das in dem Rand eines medizinischen Beutels eingearbeitet werden kann, z. B. durch Einschweißen. Das System gemäß dem B. Braun-Patent offenbart einen getrennten Zuspritz- und einen Entnahmeport. Diese Zuspritz- und Entnahmeports sind beide mit Verschlüssen versehen, um die Unversehrtheit des Beutels im Auslieferungszustand zu dokumentieren. Darüber hinaus ist es möglich, den Zuspritzport nach Zugabe einer Substanz fest zu verschließen, so dass er nicht zerstörungsfrei wieder geöffnet werden kann. Dadurch wird das versehentliche, mehrmalige Zugeben von Substanzen verhindert. Der Entnahmeport weist eine Öffnung auf, die ausschließlich mit einem passenden Spike des Herstellers kompatibel ist.

Die internationale Patentanmeldung WO 01/41698 A2 der Fresenius KABI Deutschland GmbH offenbart ebenfalls ein kombiniertes Zuspritz- und Entnahmesystem für einen medizinischen Beutel. Dieses kombinierte Zuspritz- und Entnahmesystem weist einen Zuspritzkanal und einen Entnahmekanal auf, die beide durch jeweils eine Membran verschlossen sind. Ferner umfasst das kombinierte Zuspritz- und Entnahmesystem eine dritte Öffnung, die zum Befüllen des Beutels dient und nach erfolgtem Befüllen verschlossen wird. Zuspritz- und Entnahmekanal zusammen mit dem Befüllungsstutzen sind Teil eines Basisteils in Form eines Schiffchens, das in einen Rand des medizinischen Beutels eingeschweißt werden kann.

Ferner beziehen sich die DE 10 2004 051 300 B3, die DE 102 05 344 A1 und die EP 1 063 956 B1 auf Behälter, die mittels einer Verschlusskappe verschlossen werden können.

Die Anmeldung DE 1 856 621 U offenbart eine Verschlussstopfen für Infusionsflaschen zum Durchstechen mit nadelspitz auslaufenden Übertragungsgeräten in einem Ansatzstutzen ohne Zuspritzport.

### Zusammenfassung der Erfindung

Die vorliegende Erfindung offenbart eine Verschlusskappe, die ein kombiniertes Zuspritz- und Entnahmesystem zur Verwendung mit einem medizinischen Behälter, z.B. einem Beutel oder einer Flasche umfasst. Im Folgenden wird der Begriff "medizinischer Behälter" als Synonym für "medizinischer Beutel" und "medizinische Flasche" verwendet. Im Gegensatz zum Stand der Technik ist die erfindungsgemäße Verschlusskappe aus einem harten Kunststoff gefertigt, wie er z.B. für medizinische Flaschen verwendet wird. Daher kann das Zuspritz- und Entnahmesystem direkt mit dem medizinischen Behälter verschweißt werden, was dessen Abdichtung erheblich erleichtert.

Die Verschlusskappe ist einstückig aus hartem Kunststoff ausgeführt. Der Entnahmeport ist als sich verjüngender, ins Innere des Beutels weisender Konus ausgeführt, der mindestens eine Sollbruchstelle aufweist. Der Zuspritzport ist als ins Innere des Beutels weisender Zylinder ausgeführt, der mit einer Dichtung verschlossen wird. Als Dichtung kommen Materialien z.B. Gummi in Frage, wie sie dem Fachmann bekannt sind. Die Verschlusskappe gemäß der vorliegenden Erfindung weist im Gegensatz zum Stand der Technik kein Dichtungselement im Entnahmeport auf. Das Fehlen solcher Dichtungselemente vereinfacht die Herstellung der Verschlusskappe erheblich.

Die erfindungsgemäße Verschlusskappe weist, wie bereits erwähnt, eine Membran an beiden Ports auf, die im Zuspritzport beispielsweise den permanenten Kontakt zwischen einem Dichtungselement, das z. B. aus einem Gummiwerkstoff gebildet ist, und dem Beutelinhalt verhindert. Die Gummiwerkstoffe sind im Gegensatz zu den hier eingesetzten Kunststoffen nicht inert, d.h. es kann zu einer Wechselwirkung zwischen den Gummiwerkstoffen und dem Beutelinhalt kommen. Aus diesem Grunde muss man sehr hochwertige Gummiwerkstoffe einsetzen, insbesondere wenn keine Trennmembran aus Kunststoff dazwischen liegt. Somit wird das Produkt günstiger, wenn möglichst wenig Gummimaterialien verwendet werden, und die Gummimaterialien darüber hinaus nicht der allerhöchsten Qualitätsstufe entsprechen müssen. Die erfindungsgemäße Verschlusskappe umfasst auf dem Entnahmeport kein Gummimaterial und ist daher günstig herzustellen.

Die erfindungsgemäße Ausführung des Entnahmeports als sich verjüngender, ins Innere des Behälters ragender Konus, der mindestens eine Sollbruchstelle aufweist, erlaubt insbesondere die Verwendung von unterschiedlichen Spikes zum Öffnen und zur Entnahme von Inhalt aus dem medizinischen Behälter. Darüber hinaus ist die Ausführung des Entnahmeports als Konus aus hartem Kunststoff günstiger als die Verwendung von Gummimaterialien zu Dichtungszwecken.

Im Auslieferungszustand ist der Zuspritz- und/oder der Entnahmeport mit einer Schutzfolie überzogen, die abgerissen werden kann. Die Schutzfolie zeigt die Unversehrtheit des Inhalts des Behälters an, was insbesondere ein versehentliches, mehrmaliges Zuspritzen von Substanzen zum Inhalt des Behälters verhindert. Darüber hinaus kann der Entnahmeport gemäß der Erfindung so ausgeführt werden, dass nach einem erfolgten Öffnen des Entnahmeports mittels des Spikes kein Verschließen des Entnahmeports mehr möglich ist. Es muss stattdessen der gesamte Beutelinhalt entnommen werden. Die Schutzfolie zeigt überdies eine bereits erfolgte Entnahme oder Beschädigung des Entnahmeports an.

Darüber hinaus dient die Schutzfolie auch als Kontaminationsschutz. Das Zuspritz- und/oder Entnahmesystem hat keinen Außenkontakt, so lange die Schutzfolie den Zuspritz- und/oder Entnahmeport bedeckt. Das heißt, nach erfolgter Sterilisation der Verschlusskappe ist der Zuspritz- und/oder der Entnahmeport bis zur Anwendung steril.

Die Abrissfolie ist so ausgeführt, dass zuverlässig ein Entfernen der Schutzfolie nur über einem der Ports, das heißt über entweder dem Entnhahmeport oder dem Zuspritzport möglich ist. Der jeweils andere Port, also der Zuspritz- oder der Entnahmeport bleibt weiterhin vollständig mit Abrissfolie bedeckt. Das heißt der Zuspritz- oder Entnahmeport bleibt weiterhin steril.

Die zuverlässige Entfembarkeit eines Teils der Abrissfolie über nur einem der beiden Ports kann zum Beispiel durch eine geeignete Perforation der Abrissfolie erreicht werden. In der Praxis kommt es oft vor, dass zunächst nur der Entnahmeport geöffnet wird und erst später während des Infusionsprozesses entschieden wird, dass noch ein Medikament zugespritzt werden muss. Durch die für jeden Port getrennt entfernbare Schutzfolie bleibt der Zuspritzport bis zum Zuspritzen steril.

Die Sollbruchstelle im Bereich der Spitze des Konus ist als Kontur mit reduzierter Wandstärke ausgeführt.

Das Einführen des Spikes und ein Durchdringen der Sollbruchstelle mit dem Spike bewirken, dass mindestens ein Spleißstück den eingeführten Spike an einem Umfang eng umschließt und sicher hält.

Mindestens ein Spleißstück, das den Umfang des eingeführten Spikes eng umschließt und gegen den Inhalt des Behälters abdichtet.

Die Verschlusskappe gemäß der vorliegenden Erfindung, wobei der Konus auf seiner Innenseite eine Dichtungslippe umfasst. Die Dichtungslippe dient zum Abdichten des Spikes gegen ein Herauslaufen des Inhalts des Behälters während des Einführens des Spikes.

Die Verschlusskappe gemäß der vorliegenden Erfindung, erlaubt die Verwendung der Verschlusskappe in Verbindung mit Spikes unterschiedlicher Größe. Spikes unterschiedlicher Größe können die Sollbruchstelle öffnen und werden von mindestens einem Spleiss-Stück umschlossen und gehalten. Ferner werden durch die Erfindung unterschiedliche Spikes gegen ein Herauslaufen von Inhalt des Behälters abgedichtet.

Der Zuspritzport der Verschlusskappe ist als in das Innere des Behälters ragender Zylinder aus festem Material ausgeführt.

Die durchstochene Außenfläche des Zuspritzports ist durch eine außen liegende Dichtung zuverlässig verschließbar, nachdem die Kanüle entfernt ist.

Die Verschlusskappe gemäß der vorliegenden Erfindung kann einstückig auf einer Bodenplatte ausgeführt sein.

Die Bodenplatte weist in einem Randbereich einen Wulst oder Rippen auf. Dadurch lässt sich das für die Herstellung der Verschlusskappe benötigte Material reduzieren.

Die Erfindung betrifft auch ein Verfahren zur Entnahme eines Inhalts aus einem Behälter, welches folgende Schritte umfasst:
- Einführen eines Spikes in den Konus des Entnahmeports;
- Durchstechen mindestens einer Sollbruchstelle, die im Bereich der Spitze des Konus vorgesehen sind;
- Entnehmen des Inhalts des Behälters durch eine Öffnung im Spike über einen Kontakt der Öffnung mit dem Inhalt des Behälters.

Die im Rahmen dieser Erfindung offenbarte Verschlusskappe kann zusammen mit medizinischen Behältern verwendet werden.

Zeichnungen
Fig. 1 zeigt eine Ansicht einer Verschlusskappe gemäß der vorliegenden Erfindung von vorne.
Fig. 2 zeigt eine Draufsicht auf die Verschlusskappe gemäß der vorliegenden Erfindung.
Fig. 3 zeigt die Verschlusskappe in Verbindung mit einem medizinischen Behälter, wobei der Konus durch einen Spike geöffnet ist und der Spike vollständig in den Konus eingeführt ist.
Fig. 4 zeigt einen vollständig eingeführten Spike im Konus des Entnahmeports in einer Detailansicht.
Fig. 5 zeigt den Entnahmeport mit eingeführter Kanüle.

### Detaillierte Beschreibung der Erfindung

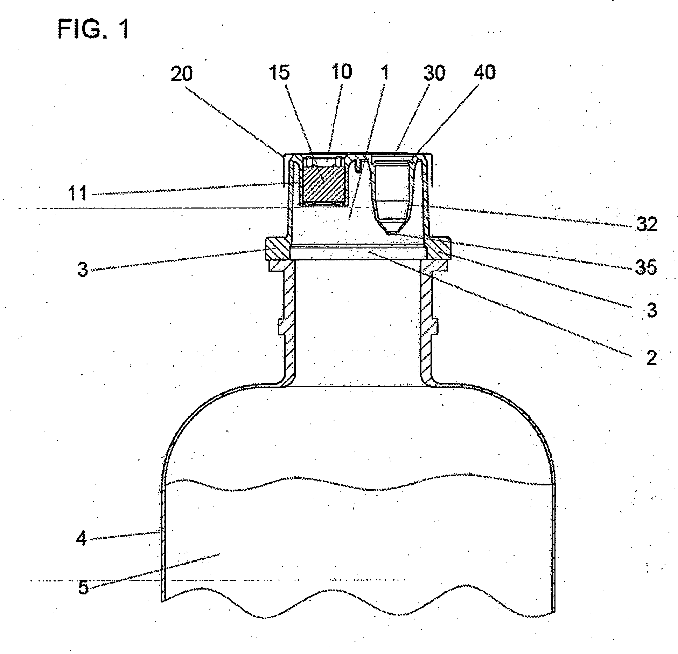

Fig. 1 zeigt eine Verschlusskappe 1 gemäß der vorliegenden Erfindung. Die Verschlusskappe 1 umfasst eine Bodenplatte 2, in deren Randbereich 3 das Material verstärkt ist, z.B. als Wulst oder Rippen. Die Boderiplatte 2 oder der Randbereich 3 wird mit einem medizinischen Behälter 4, z.B. einem Beutel oder einer Flasche verbunden. Oberhalb der Bodenplatte 2 befindet sich ein Entnahmeport 30, der als zum Behälter 4 hinweisender Konus 32 ausgebildet ist, der ins Innere der Verschlusskappe 1 ragt. Im Bereich der Spitze 35 des Konus 32 befindet sich mindestens eine Sollbruchstelle 35. In einem Randbereich des Konus 32 befindet sich eine Dichtungslippe 40, die als ringförmiges gerades Stück mit dem Konus 32 verbunden ist und vom Inhalt 5 des Behälters 4 nach außen weist. Dabei ragt die Dichtungslippe 40 nicht über den Rand des Entnahmeports 30 hinaus.

Ferner umfasst die Verschlusskappe 1 einen Zuspritzport 10, der als ein zum Behälter 4 hin weisender Zylinder 11 ausgeführt ist. Der so entstandene Hohlraum auf der Außenseite des Zuspritzports 10 ist mit einem Dichtungsmaterial 15 angefüllt. Ferner zeigt Fig. 1 die Verschlusskappe 1 in einem Auslieferungszustand. Sowohl der Entnahmeport 30 als auch der Zuspritzport 10 sind mit einer Abreißfolie 20 bedeckt. Es ist ohne Einschränkung möglich, dass in einem Auslieferungszustand der Verschlusskappe 1 lediglich der Zuspritzport 10 mit Abreißfolie 20 bedeckt ist.

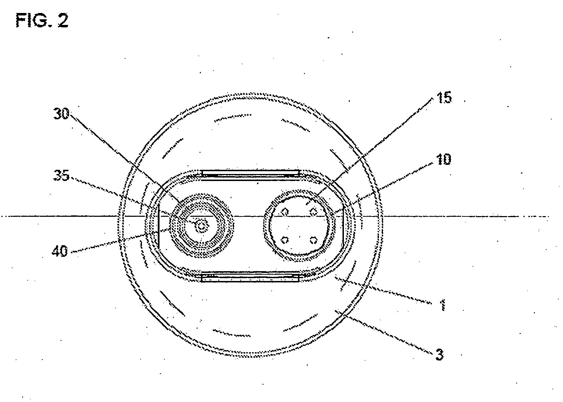

Fig. 2 zeigt eine Draufsicht der Verschlusskappe 1 gemäß der Erfindung. Gut zu erkennen sind auch hier der Zuspritzport 10, der mit dem Dichtungsmaterial 15 angefüllt ist, sowie der Entnahmeport 30, der in einer Spitze zuläuft. Der Randbereich 3 der Bodenplatte 2 ist verstärkt ausgeführt, z.B. als Wulst oder Rippe, was ebenfalls gut zu erkennen ist.

Die Verschlusskappe 1 gemäß der Erfindung ist aus einem festen Material gefertigt, z. B. PE. Die Wandstärke der Verschlusskappe 1 liegt generell im Bereich von 0,2 - 1 mm; im Durchstichbereich hingegen im Bereich von 0,2 - 0,6 mm. Dieser Wert stellt allerdings keine Einschränkung der Erfindung dar, vorausgesetzt, die Wandstärken des Zuspritzports 10 und des Entnahmeports 30 sind nicht zu stark, um mit einer Kanüle 60 oder einem Spike 50 geöffnet zu werden. Die Verschlusskappe 1 wird durch ein geeignetes Verfahren z.B. Verschweißen mit dem Behälter 4 verbunden, so dass ein direkter Kontakt mit dem Inhalt 5 des Behälters 4 entsteht. Die Verschlusskappe 1 kann dazu in den Rand eines aus Folienmaterial bestehenden Beutels eingearbeitet werden. Darüber hinaus ist es möglich, die Verschlusskappe 1 direkt mit einem flächigen Anteil des Beutels zu verbinden, etwa durch ein geeignetes Schweißverfahren oder unter Verwendung eines geeigneten Adapterstücks, das mit dem Beutel verbunden ist. Ferner ist es möglich, die Verschlusskappe 1 zusammen mit einer medizinischen Flasche zu verwenden, wobei die Verschlusskappe 1 unmittelbar an die Flasche anschließt. Vorzugsweise könnte die Verschlusskappe 1 in einer solchen Ausführungsform an den Hals der Flasche anschließen. Das für die erfindungsgemäße Verschlusskappe 1 verwendete Kunststoffmaterial ist relativ hart. Das Kunststoffmaterial kann dem für die medizinische Flasche verwendeten Material entsprechen, was die Herstellung der Verschlusskappe als Teil einer medizinischen Flasche weiter vereinfacht. Selbstverständlich ist es möglich, einen geeigneten Kunststoff zu verwenden, der sich von dem Material, aus dem die Flasche gefertigt ist, unterscheidet.

Wie im Folgenden ausgeführt wird, ist die mechanische Festigkeit des Materials, zumindest für den Konus 32 nützlich für das zweckmäßige Funktionieren der Verschlusskappe 1 gemäß der vorliegenden Erfindung.

Der Entnahmeport 30 der Verschlusskappe 1 ist als ins Innere des Behälters 4 weisender Konus 32 ausgeführt. Insbesondere weist der Konus 32 im Bereich seiner Spitze mindestens eine Sollbruchstelle 35 auf. Es ist zweckmäßig, die Sollbruchstelle 35 als eine Kontur mit verminderter Wandstärke auszuführen.

Im Auslieferungszustand wird der Behälter 4 gemeinsam mit der Verschlusskappe 1 und einem therapeutischen, flüssigen Inhalt 5 an z. B. Kliniken oder Ärzte geliefert. Im Auslieferungszustand der Verschlusskappe 1 sind der Entnahmeport 30 und/oder der Zuspritzport 10 durch eine Abrissfolie 20 bedeckt. Die Abrissfolie 20 dient zur Überprüfung der Unversehrtheit der Verschlusskappe 1 und des Behälters 4. Um den Entnahmeport 30 und den Zuspritzport 10 zugänglich zu machen, muss die Abrissfolie 20 entfernt werden. Dies erfolgt praktischerweise zumindest so weit, dass beide Ports zugänglich sind.

Darüber hinaus stellt die Abrissfolie 20 einen Kontaminationsschutz dar. Der Zuspritzport 10 und/oder der Entnahmesport 30 haben keinen Außenkontakt, so lange die Abrissfolie 20 den Zuspritzport 10 und/oder den Entnahmeport 30 bedeckt. Mittels geeigneter Sterilisationsverfahren, die dem Fachmann hinreichend bekannt sind, kann die Verschlusskappe 1 sterilisiert werden. Die verwendete Abrissfolie 20 stellt sicher, dass Entnahmeport 30 und Zuspritzport 10 bis zur Verwendung der Verschlusskappe 1 steril bleiben.

In einer Ausführungsform der Verschlusskappe 1 ist es möglich, die Abrissfolie 20 in ihrer Mitte perforiert auszuführen, so dass die Abrissfolie 20 einzeln für Entnahmeport 30 oder Zuspritzport 10 entfernt werden kann, während der jeweils andere Port weiterhin von dem verbleibenden Teil der Abrissfolie 20 bedeckt bleibt. Bei der praktischen Verwendung einer Verschlusskappe 1 kommt es oft vor, dass zunächst nur der Entnahmeport 30 geöffnet wird und erst später, während des Infusionsprozesses, entschieden wird, dass noch ein Medikament zum Beutelinhalt 5 zugespritzt werden muss. Durch die getrennt entfernbare Abrissfolie 20 bleibt der Zuspritzport 10 bis zum eigentlichen Zuspritzen steril.

Sofern Substanzen zum Inhalt 5 des Behälters 4 zugegeben werden sollen, wird die Kanüle 60 in den Zuspritzport 10 eingeführt, bis sie die Dichtung und eine Außenwand des Zylinders 11 durchstochen hat. Sinnvollerweise wird für das Zuführen von Substanzen eine sterile Kanüle 60 verwendet. Eine nicht-sterile Kanüle 60 birgt für den Patienten ein Infektionsrisiko. Über die in den Zuspritzport 10 eingeführte Kanüle 60 können nun Substanzen zum Inhalt 5 des Behälters 4 zugegeben werden. Am günstigsten kann diese Zugabe etwa unter Verwendung einer Spritze erfolgen, welche die zuzuführenden Substanzen enthält.

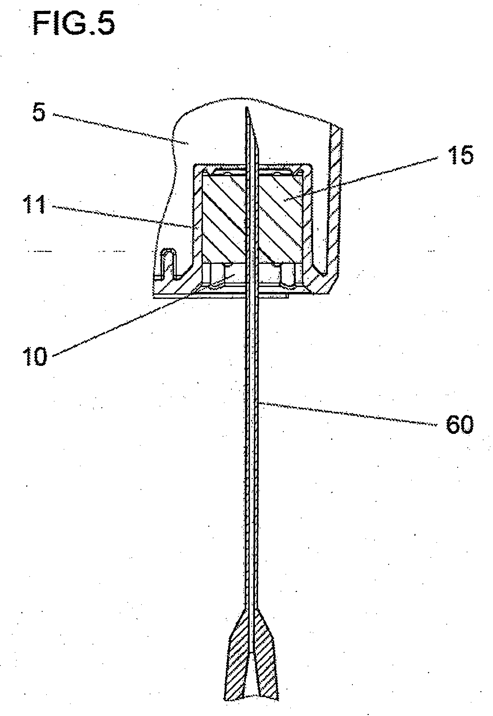

In Fig. 5 ist ein Detailausschnitt der Verschlusskappe 1 gezeigt. Eine Außen bzw. Bodenwand des Zylinders 11 des Einspritzports 10 ist dabei von der Kanüle 60 durchstochen. Wird nun die Kanüle 60 entfernt, so verbleibt ein Einstichloch in der Außenwand des Zylinders 11 des Zuspritzports 10. Das Dichtungsmaterial 15, in Fig. 5 unterhalb des Einstichlochs, dichtet das Einstichloch der Kanüle 60 hinreichend von der Umgebung ab, so dass kein Inhalt 5 des Behälters 4 über das Einstichloch nach außen gelangen kann. Das Dichtungsmaterial 15 kann, wie dem Fachmann bekannt, durch ein geeignetes Material ausgeführt sein, z.B. Gummiwerkstoffe wie Butyl oder Polyisopren, weiterhin Silikon oder thermoplastisches Elastomer (TPE).

Ein Abdichten des Einstichlochs durch das Dichtungsmaterial 15 ist erforderlich. Fehlt dieses Abdichten mittels des Dichtungsmaterials 15, so kann der Inhalt 5 des Behälters 4 über das Einstichloch der Kanüle 60 entweichen. Ferner können Verunreinigungen von außerhalb des Behälters 4 über das Einstichloch mit dem Inhalt 5 des Beutels 4 in Kontakt treten, was diesen unter Umständen verunreinigt. Ein sicheres Wiederverschließen des Einstichlochs ist daher für medizinische Anwendungen vorteilhaft.

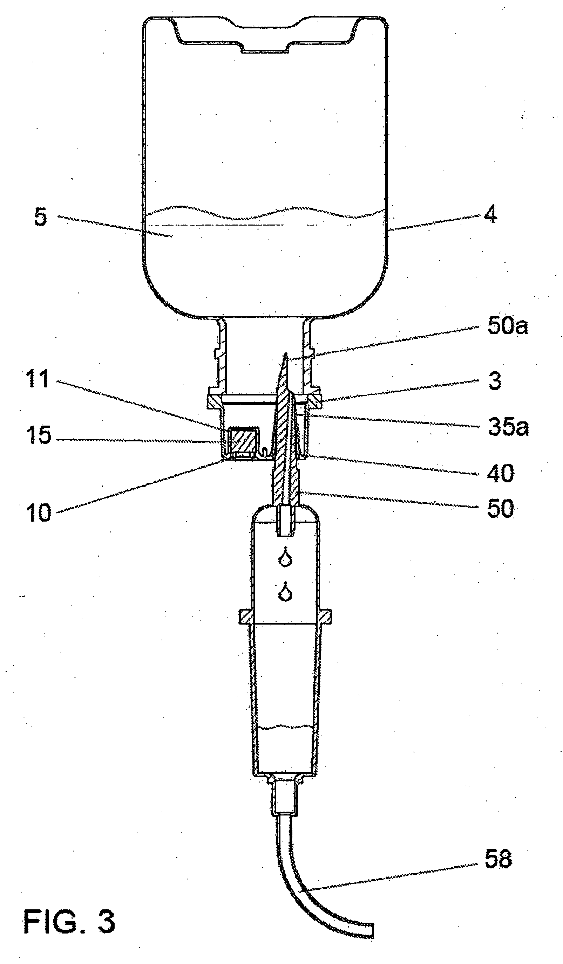

Der Entnahmeport 30 der Verschlusskappe 1 gemäß der vorliegenden Erfindung ist vorzugsweise aus festem, massiven Material ausgeführt, z.B. PE oder PP von einer Wandstärke im Bereich 0,1 - 0,6 mm, vorzugsweise 0,2 mm. Im Bereich der Spitze des Konus 32, wie z. B. in Figur 1 gezeigt, befindet sich mindestens eine Sollbruchstelle 35. Es ist günstig diese Sollbruchstelle 35 als eine Kontur reduzierter Wandstärke auszuführen. So kann die Sollbruchstelle z.B. als eine Kreuzkontur reduzierter Wandstärke ausgeführt sein. Selbstverständlich sind auch andere Formen für die Kontur reduzierter Wandstärke, welche die Sollbruchstelle 35 definiert, möglich. Die Wandstärke des Konus 32 im Bereich der Sollbruchstelle 35 muss so stark sein, dass die Wandstärke einerseits den Druck des Inhalts 5 des Behälters 4 standhält und andererseits dünn genug sein, dass sie mittels einer Spritze des Spikes 50 geöffnet werden kann. Wird nun die Spitze des Spikes 50 in die Öffnung des Entnahmeports 30 eingeführt, wie gezeigt in Figur 3, so schmiegt sich die Dichtungslippe 40 um den Spike 50. Der Spike 50 zur Entnahme kann ohne Widerstand eingeführt werden, bis die Spitze des Spikes die Sollbruchstelle 35 berührt. Bei weiterem Druck des Spikes 50 auf die Sollbruchsstelle 35 bricht die Sollbruchstelle 35 und reißt entlang der Kontur reduzierter Wandstärke auf.

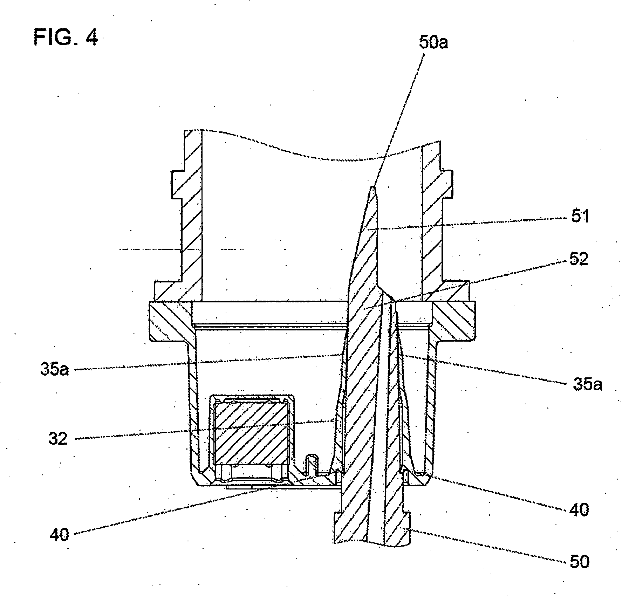

Da der Konus 32 aus festem Material gefertigt ist, spleißt die Sollbruchstelle 35 auf und legt sich gleichzeitig, aufgrund ihrer Festigkeit eng um den Anteil des Spikes 50, der die Sollbruchstelle 35 bereits durchdrungen hat. Ein Beispiel für eine aufgespleißte Sollbruchstelle 35a ist in Fig. 3 und 4 zu sehen. Dabei befindet sich die Verschlusskappe 1 in Fig. 3 in der für Infusionsflaschen und Beutel üblichen Kopf-über-Stellung.

In Fig. 4 ist ein gerader Spike 50 gezeigt, der unterhalb seiner Spitze einen Bereich mit einem Umfang 52 aufweist. Wird nun der Spike 50 so weit in den Entnahmeport 30 gedrückt, dass der Umfang 52 die aufgespleißten Spleiß-Stücke 35a der Sollbruchstelle 35 passiert, so wird dies im Rahmen dieser Beschreibung als Einführen 51 des Spikes 50 bezeichnet. Durch das Einführen 51 des Spikes 50 in den Entnahmekanal 32 ergibt sich ein definierter Zustand für den eingeführten Spike 50, wie er für medizinische Anforderungen gebräuchlich ist.

Zum einen halten Spleiß-Stücke 35a der aufgespleißten Sollbruchstelle 35 aufgrund ihrer hohen Festigkeit den Spike 50 fest umschlossen.

Das für den erfindungsgemäßen Konus 32 gewählte Material, die Geometrie der Sollbruchstelle 35 sowie die Wandstärke des Konus 32 sind so gewählt, dass der Spike 50 nach Einführen des Spikes 50 über 4 Stunden ein Gewicht von 1 kg tragen kann, wie es durch entsprechende Vorschriften gefordert ist, z. B. ISO 15759.

Ferner liegen die Spleiss-Stücke 35a der aufgebrochenen Sollbruchsteile 35, wiederum aufgrund der hohen Festigkeit des Materials sowie der gewählten Wandstärke der Kontur der Sollbruchstelle 35, eng an dem reduzierten Umfang 52 des Spikes 50. So wird der eingeführte Spike 50 sicher gegen den Inhalt 5 des Behälters 4 abgedichtet.

Es sind neben geraden Spikes 50 auch solche mit einer Taillierung unterhalb der Spitze gebräuchlich. Die in Fig. 3 und 4 gezeigte Form eines geraden Spikes stellt keine Einschränkung der Erfindung dar. Auch in Verbindung mit Spikes 50 mit einer Taillierung unterhalb der Spitze kann der Entnahmeport 30 erfindungsgemäß verwendet werden. Die Wandstärke der Sollbruchstelle 35, die Festigkeit des verwendeten Materials und die Kontur der Sollbruchstelle 35 können so gewählt werden, dass die Spleiß-Stücke 35a bei Einführen 51 des Spikes 50 hart genug sind, um über 4 Stunden ein Gewicht von 1 kg am Spike 50 zu tragen und zwar für gerade Spikes 50 ebenso wie taillierte Spikes.

Durch Einführen 51 des Spikes 50 erzeugt eine Öffnung 50a des Spikes 50 einen Kontakt zwischen dem Inhalt 5 des Behälters 4 und einem Schlauchsystem 58, wie in Figur 3 gezeigt. Für die in Fig. 3 und 4 gezeigte Ausführungsform ist die Öffnung 50a als Teil einer Hohlnadel ausgebildet. Solch eine Form von Spikes 50 ist gebräuchlich. Die genaue Form des Kontakts zwischen dem Inneren des Behälters 4 und einem Außenbereich des Behälters 4 über eine Öffnung 50a des Spikes ist für das Funktionieren der Verschlusskappe 1 gemäß der Erfindung nicht von Belang. Es genügt, dass dieser Kontakt von Inhalt 5 des Behälters 4 und dem Außenbereich des Behälters 4 über eine Öffnung 50a des Spikes 50 möglich ist.

Das Schlauchsystem 58 ermöglicht einen Kontakt zu einem Patienten z.B. einen Zugang zu einer Vene des Patienten.

Ein bloßer Kontakt zwischen dem Inhalt 5 des Behälters 4 und der Öffnung 50a des Spikes 50 ist bereits mit dem Durchbrechen der Sollbruchstelle 35 erreicht. Ab diesem Zeitpunkt kann Inhalt 5 aus dem Behälter 4 über die Öffnung 50a heraus fließen.

Um das Herauslaufen von Inhalt 5 des Behälters 4 ab dem Durchbrechen der Sollbruchstelle 35 zu verhindern, ist die Dichtungslippe 40 vorgesehen, wie in Fig. 3 und 4 gut zu erkennen. Die Dichtungslippe 40 schmiegt sich eng an den Umfang des Spikes 50, so dass keine Flüssigkeit mehr austreten kann. Im Gegensatz zur Wand des Konus 32 ist die Dichtungslippe 40 relativ flexibel ausgeführt, was sich am einfachsten durch eine geringere Wandstärke erreichen lässt. Selbstverständlich ist es ebenfalls möglich, die Dichtungslippe 40 aus einem anderen Material zu formen.

Das Vorsehen der Dichtungslippe 40 verhindert einen unkontrollierten Kontakt zwischen dem Inhalt 5 des Behälters 4 und einem Bereich außerhalb der Verschlusskappe 1. Solch eine strikte Trennung beider Bereiche ist nützlich, um Kontaminationen des Inhalts 5 zu vermeiden, und /oder Inhalt 5 des Behälters 4 unkontrolliert abzugeben, ohne dass der Inhalt 5 in das Schlauchsystem 58 gelangt. Solch ein unkontrollierter Kontakt stellt für den Patienten ein Risiko dar, wie bereits weiter oben beschrieben worden ist.

Aus produktionstechnischen Gründen kann es günstig sein, die gesamte Verschlusskappe 1 aus dem festen Material herzustellen, das für den Konus 32 verwendet wird. Dadurch würde die Herstellung der Verschlusskappe 1 der vorliegenden Erfindung zusätzlich erleichtert. Die Ausbildung des Konus 32 und der Sollbruchstelle 35 ist insbesondere so zu wählen, dass bei dem Einführen 51 des Spikes 50 der Spike 50 von den Spleißstücken 35a gehalten wird und gegen den Inhalt 5 des Behälters 4 abgedichtet ist.

Im klinischen Bereich sind Spikes 50 gebräuchlich, die sich hinsichtlich ihrer Abmessungen und ihrer Geometrien unterscheiden.

Der sich verjüngende, ins Innere des Behälters 4 ragende Konus 32 gemäß der vorliegenden Erfindung erlaubt die Verwendung der Verschlusskappe 1 in Verbindung mit unterschiedlichen Spikes 50. Dies ist gewährleistet auf Grund der gewählten Geometrie des Konus 32, der Wandstärke des Konus 32, der Kontur der Sollbruchstellen 35 sowie den Abmessungen und Wandstärken der Dichtungslippe 40.

Durch diese Eigenschaft der erfindungsgemäßen Verschlüsskappe 1 vereinfacht sich der klinische Einsatz medizinischer Behälter 4 erheblich, da nicht mehr der eine, mit der verwendeten Verschlusskappe 1 kompatible, Spike 50, gesucht werden muss.

Die im Rahmen dieser Offenlegung beschriebene Verschlusskappe 1 für einen medizinischen Behälter 4, z.B. einen medizinischen Beutel oder eine medizinische Flasche ist geeignet für den Einsatz im medizinisch-klinischen Umfeld und erleichtert den Umgang mit medizinischen Flüssigkeiten die Patienten verabreicht werden sollen, erheblich.

Der Einsatz der Verschlusskappe 1 gemäß der vorliegenden Erfindung ist nicht auf Systeme zur intravenösen Verabreichung von therapeutischen Flüssigkeiten beschränkt. Ohne Einschränkung lässt sich die Verschlusskappe 1 für jede medizinischklinische Anwendung verwenden, in der ein kontrollierter Übergang von einem Behälter 4 zum Patienten nötig ist, z.B. der Verabreichung von Produkten zur künstlichen Ernährung.

## Patentansprüche

1. Eine Verschlusskappe (1) für einen medizinischen Behälter (4), wobei die Verschlusskappe (1) einen Zuspritzport (10) zur Beimischung von Substanzen zum Inhalt (5) des Behälters (4) sowie einen Entnahmeport (30) zur Entnahme des Inhalts (5) des Behälters (4) umfasst, wobei der Entnahmeport (30) konfiguriert ist, um in einen Innenraum des Behälters (4) zu weisen, und als ein sich verjüngender Konus (32) ausgeführt ist, der mindestens eine Sollbruchstelle (35) im Bereich der Spitze des Konus (32) aufweist, **dadurch gekennzeichnet, dass** der Konus (32) auf seiner Innenseite eine Dichtungslippe (40) umfasst.

2. Die Verschlusskappe (1) nach Anspruch 1, wobei der Konus (32) aus hinreichend festem Material gefertigt ist und die Sollbruchstelle (35) durch das Einführen des Spikes (50) öffenbar ist, so dass ein Kontakt zwischen dem Inhalt (5) des Behälters (4) und einer Öffnung in dem Spike (50) zur Entnahme des Inhalts (5) entsteht.

3. Verschlusskappe (1) nach Anspruch 1 oder 2, wobei die Sollbruchstelle (35) als eine Kontur mit reduzierter Wandstärke ausgebildet ist.

4. Die Verschlusskappe (1) nach einem der vorangegangenen Ansprüche, wobei das Einführen (51) des Spikes (50) und ein Durchdringen der Sollbruchstelle (35) mit dem Spike (50) bewirken, dass mindestens ein Spleiss-Stück (35a) den eingeführten Spike an einem Umfang (52) eng umschließt und sicher hält.

5. Die Verschlusskappe (1) nach Anspruch 4, wobei das mindestens eine Spleiss-Stück (35a) den Umfang (52) des eingeführten Spikes (50) eng umschließt und so gegen den Inhalt (5) des Behälters (4) abdichtet.

6. Die Verschlusskappe (1) nach einem der vorangegangenen Ansprüche, wobei die Sollbruchstelle (35) des Konus (32) durch die Spikes (50) unterschiedlicher Größe durchdringbar ist.

7. Die Verschlusskappe (1) nach einem der vorangegangenen Ansprüche, wobei der Zuspritzport (10) als in das Innere des Behälters (4) weisender Zylinder (11) aus festem Material ausgeführt ist.

8. Die Verschlusskappe (1) nach einem der vorigen Ansprüche, wobei eine Außenfläche des Zuspritzports (10) durch eine Kanüle (60) durchstechbar ist, was die Zugabe von Substanzen zum Inhalt (5) des Beutels (4) erlaubt.

9. Die Verschlusskappe (1) nach Anspruch 8, wobei die durchstochene Außenfläche des Zuspritzports (10) durch eine Dichtung zuverlässig verschließbar nach der Entfernung der Kanüle (60) ist.

10. Die Verschlusskappe (1) nach Anspruch 9, wobei die Dichtung aus einem geeigneten Dichtungsmaterial (15) ausgeführt ist, das aus der Gruppe bestehend aus Butylisopren, Polyisopren, Silikon, Gummi oder thermoplastische Elastomere (TPE) ausgewählt werden kann.

11. Die Verschlusskappe (1) nach einem der vorhergehenden Ansprüche, wobei zumindest der Zuspritzport (10) und/oder der Entnahmeport (30) in einem Auslieferungszustand der Verschlusskappe (1) mit einer Abrissfolie (20) bedeckt sind.

12. Die Verschlusskappe (1) gemäß Anspruch 11, wobei die Abrissfolie (20) auf dem Zuspritzport (10) oder dem Entnahmeport (30) einzeln entfernbar ist, und der verbleibende Teil der Abrissfolie (20) jeweils den Entnahmeport (30) oder den Zuspritzport (10) weiterhin steril hält.

13. Die Verschlusskappe (1) nach einem der vorangegangenen Ansprüche, wobei die Verschlusskappe (1) als einstückig auf einer Bodenplatte (2) ausgeführt ist.

14. Die Verschlusskappe nach Anspruch 13, wobei die Bodenplatte (2) in einem Randbereich (3) einen Wulst oder Rippen aufweist.

15. Verfahren zur Entnahme eines Inhalts (5) aus einem medizinischen Behälter (4), wobei der Behälter (4) eine Verschlusskappe (1) mit einem Zuspritzport (10) zur Beimischung von Substanzen zum Inhalt (5) des Behälters (4) und einem als in den Innenraum des Behälters (4) weisenden, sich verjüngenden Konus (32) ausgebildeten Entnahmeport (30) zur Entnahme des Inhalts (5) des Behälters (4) aufweist, wobei das Verfahren folgende Schritte umfasst:
- Einführen (51) eines Spikes (50) in den Konus (32) des Entnahmeport (30);
- Durchstechen mindestens einer Sollbruchstelle (35), die im Bereich der Spitze des Konus (32) vorgesehen ist, mit dem Spike (50);
- Entnehmen des Inhalts (5) des Behälters (4) durch eine Öffnung im Spike (50) über einen Kontakt der Öffnung mit dem Inhalt (5) des Behälters (4), das Verfahren weiterhin umfassend das Verhindern eines Auslaufens des Inhaltes (5) des Behälters (4) beim Einführen des Spikes (50) durch das Abdichten des Spikes (50) mittels einer an der Innenfläche des Konus (32) angeordneten Dichtungslippe (40).

16. Das Verfahren nach Anspruch 15, weiter umfassend das Umschließen des eingeführten Spikes (50) durch mindestens ein Spleiss-Stück (35a).

17. Das Verfahren gemäß Anspruch 15 oder 16, weiter umfassend das Abdichten des Spikes (50) gegen den Inhalt (5) des Behälters (4) durch das Spleiss-Stück (35a).

## Claims

1. A closure cap (1) for a medical container (4), wherein the closure cap (1) has an injection port (10) for admixing substances to the content (5) of the container (4) and an extraction port (30) for extracting the content (5) of the container (4), wherein the extraction port (30) is configured to be directed towards an interior of the container (4), and is executed as a tapered cone (32) having at least one predetermined breaking point (35) in the area of the tip of the cone (32), **characterized in that** the cone (32) comprises a sealing lip (40) on its inner side.

2. The closure cap (1) according to claim 1, wherein the cone (32) is made of sufficiently solid material and the predetermined breaking point (35) is openable by inserting the spike (50), such that a contact between the content (5) of the container (4) and an opening in the spike (50) is formed for extracting the content (5).

3. The closure cap (1) according to claim 1 or 2, wherein the predetermined breaking point (35) is formed as a contour with a reduced wall thickness.

4. The closure cap (1) according to any of the preceding claims, wherein the insertion (51) of the spike (50) and a penetration of the predetermined breaking point (35) with the spike (50) cause at least one splicing element (35a) to closely surround and securely hold the inserted spike on a circumference (52).

5. The closure cap (1) according to claim 4, wherein the at least one splice element (35a) closely surrounds the circumference (52) of the inserted spike (50), thus sealing it against the content (5) of the container (4).

6. The closure cap (1) according to any of the preceding claims, wherein the predetermined breaking point (35) of the cone (32) can be penetrated by the spikes (50) of different sizes.

7. The closure cap (1) according to any of the preceding claims, wherein the injection port (10) is executed as a cylinder (11) of solid material being directed towards the interior of the container (4).

8. The closure cap (1) according to any of the preceding claims, wherein an outer surface of the injection port (10) can be pierced by a cannula (60), permitting the addition of substances to the content (5) of the bag (4).

9. The closure cap (1) according to claim 8, wherein the pierced outer surface of the injection port (10) can be reliably closed by a seal after removal of the cannula (60).

10. The closure cap (1) according to claim 9, wherein the seal is executed of a suitable sealing material (15), which can be chosen from the group consisting of butyl isoprene, polyisoprene, silicone, rubber, or thermoplastic elastomers (TPE).

11. The closure cap (1) according to any of the preceding claims, wherein at least the injection port (10) and/or the extraction port (30) are covered by a tear-off foil (20) in a delivery state of the closure cap (1).

12. The closure cap (1) according to claim 11, wherein the tear-off foil (20) on the injection port (10) or the extraction port (30) is individually removable, and the remaining part of the tear-off foil (20) continues to keep the corresponding extraction port (30) or injection port (10) sterile.

13. The closure cap (1) according to any of the preceding claims, wherein the closure cap (1) is executed integrally on a base plate (2).

14. The closure cap according to claim 13, wherein the base plate (2) has a bead or ribs in a peripheral area (3).

15. A method for extracting a content of (5) from a medical container (4), wherein the container (4) has a closure cap (1) with an injection port (10) for admixing substances to the content (5) of the container (4) and an extraction port (30) formed as a tapered cone (32) being directed towards the interior of the container (4) for extracting the content (5) of the container (4), wherein the method comprises the following steps of:
- inserting (51) a spike (50) into the cone (32) of the extraction port (30);
- piercing with the spike (50) at least one predetermined breaking point (35) provided in the area of the tip of the cone (32);
- extracting the content (5) of the container (4) through an opening in the spike (50) via a contact of the opening with the content (5) of the container (4), the method further comprising preventing a leaking of the content (5) of the container (4) upon insertion of the spike (50) by sealing the spike (50) by means of a sealing lip (40) arranged on the inner surface of the cone (32).

16. The method of claim 15, further comprising surrounding the inserted spike (50) by at least one splice element (35a).

17. The method according to claim 15 or 16, further comprising sealing the spike (50) against the content (5) of the container (4) by the splice element (35a).

## Revendications

1. Bouchon de fermeture (1) pour un récipient médical (4), dans lequel le bouchon de fermeture (1) comprend un port d'injection (10) pour mélange de substances pour former le contenu (5) du récipient (4) ainsi qu'un port de sortie ou de prélèvement (30) pour prélèvement du contenu (5) du récipient (4), dans lequel le port de prélèvement (30) est configuré pour être orienté vers un espace interne du récipient (4) et est conçu en forme de cône se rétrécissant (32), comprenant au moins une zone de rupture (35) dans le domaine de la pointe du cône (32), **caractérisé en ce que** le cône (32) comprend une lèvre d'étanchéité (40) sur son côté interne.

2. Bouchon de fermeture (1) selon la revendication 1 dans lequel le cône (32) est fabriqué d'un matériau suffisamment solide, et la zone de rupture (35) peut être ouverte par l'introduction d'une pointe (50), de sorte qu'un contact est établi entre le contenu (5) du récipient (4) et une ouverture dans la pointe (50) pour prélèvement du contenu (5).

3. Bouchon de fermeture (1) selon la revendication 1 ou 2, dans lequel a zone de rupture (35) est formée en tant que contour à épaisseur de paroi réduite.

4. Bouchon de fermeture (1) selon l'une quelconque des revendications précédentes, dans lequel l'introduction (51) de la pointe (50) et une traversée de la zone de rupture (35) avec la pointe (50) agissent de telle sorte qu'au moins un morceau d'épissure (35a) entoure et maintient fermement la pointe introduite sur une périphérie (52).

5. Bouchon de fermeture (1) selon la revendication 4, dans lequel la au moins une partie d'épissure (35a) entoure étroitement la périphérie (52) de la pointe introduite (50) et étanchéifie ainsi contre le contenu (5) du récipient (4).

6. Bouchon de fermeture (1) selon l'une quelconque des revendications précédentes, dans lequel la zone de rupture (35) du cône (32) peut être traversée par des pointes (50) de taille différente.

7. Bouchon de fermeture (1) selon l'une quelconque des revendications précédentes, dans lequel le port d'injection (10) est réalisé en tant que cylindre (11) projetant à l'intérieur du récipient (4), réalisé en un matériau solide.

8. Bouchon de fermeture (1) selon l'une quelconque des revendications précédentes, dans lequel une surface du port d'injection (10) peut être percée par une canule (60), ce qui permet l'apport de substances dans le contenu (5) de la poche (4).

9. Bouchon de fermeture (1) selon la revendication 8, dans lequel la surface percée du port d'injection (10) peut être refermée par un joint d'étanchéité fiable après le retrait de la canule (60).

10. Bouchon de fermeture (1) selon la revendication 9, dans lequel le joint d'étanchéité est fait d'un matériau d'étanchéité (15) adapté, qui est choisi dans le groupe constitué par le butylisoprène, polyisoprène, silicone, caoutchouc ou élastomère thermoplastique (TPE).

11. Bouchon de fermeture (1) selon l'une quelconque des revendications précédentes, dans lequel au moins le port d'injection (10) et/ou le port de prélèvement (30) est recouvert avec une feuille déchirable (20), dans un état de livraison du bouchon de fermeture (1).

12. Bouchon de fermeture (1) selon la revendication 11, dans lequel la feuille déchirable (20) peut être retirée individuellement du port d'injection (10) ou sur le port de prélèvement (30), et la partie restante de la feuille déchirable (20) continue de maintenistérile le port d'injection (10) ou le port de prélèvement (30), respectivement.

13. Bouchon de fermeture (1) selon l'une quelconque des revendications précédentes, dans lequel le bouchon de fermeture (1) est réalisé en tant que pièce intégrale sur une plaque de fond (2).

14. Bouchon de fermeture selon la revendication 13, dans lequel la plaque de fond (2) comprend des nervures ou bourrelets dans un domaine de rebord (3).

15. Procédé de prélèvement d'un contenu (5) d'un récipient médical (4), dans lequel le récipient (4) comprend un bouchon de fermeture (1) avec un port d'injection (10) pour mélange de substances dans le contenu (5) du récipient (4) et un port de prélèvement (30) formé en tant que cône se rétrécissant (32) et orienté vers l'intérieur du récipient (4), pour prélèvement du contenu (5) du récipient (4), dans lequel le procédé comprend les étapes suivantes :
- introduction (51) d'une pointe (50) dans le cône (32) du port de prélèvement (30) ;
- perçage d'au moins une zone de rupture (35), prévue dans le domaine de pointe du cône (32), à l'aide la pointe (50) ;
- prélèvement du contenu (5) du récipient (4) à travers une ouverture dans la pointe (50) par l'intermédiaire d'un contact de l'ouverture avec le contenu (5) du récipient (4), le procédé comprenant en outre l'étape consistant à empêcher une fuite du contenu (5) du récipient (4) lors de l'introduction de la pointe (50) par l'étanchéisation de la pointe (50) au moyen d'une lèvre d'étanchéité (40) agencée sur la surface interne du cône (32).

16. Procédé selon la revendication 15, comprenant en outre l'étape d'entourer la pointe introduite (50) par l'intermédiaire d'au moins un morceau d'épissure (35a).

17. Procédé selon la revendication 15 ou 16, comprenant en outre l'étape d'étanchéifier la pointe (50) contre le contenu (5) du récipient (4) par l'intermédiaire du morceau d'épissure (35a).
